(19) Europäisches Patentamt
European Patent Office
Office européen des brevets

(11) Veröffentlichungsnummer: **0 212 281**
**B1**

# (12) EUROPÄISCHE PATENTSCHRIFT

(45) Veröffentlichungstag der Patentschrift: **06.09.89**

(51) Int. Cl.[4]: **C 07 D 401/04**, C 07 D 403/04, A 01 N 43/56

(21) Anmeldenummer: **86109958.8**

(22) Anmeldetag: **21.07.86**

## (54) 1-Heteroaryl-4-aryl-pyrazol-Derivate.

(30) Priorität: **30.07.85 DE 3527157**

(43) Veröffentlichungstag der Anmeldung:
**04.03.87 Patentblatt 87/10**

(45) Bekanntmachung des Hinweises auf die Patenterteilung:
**06.09.89 Patentblatt 89/36**

(84) Benannte Vertragsstaaten:
**AT BE CH DE FR GB IT LI NL**

(56) Entgegenhaltungen:
**EP-A- 0 165 448**
**EP-A- 0 183 159**
**DE-A- 2 651 008**

**CHEMICAL ABSTRACTS, Band 70, Nr. 3, 20 Januar 1969, Columbus, Ohio, USA GRANDBERG, I.I.; KROKHINA, N.F.; KONDRAT'EV, M.N.:"Pyrazoles. LXV. Synthesis of a series of 5-hydroxy- and 5-aminopyrazoles with nitrogen-containing functional substituents" Seite 279, Spalte 2, Zusammenfassung-Nr. 11 624c**
**CHEMICAL ABSTRACTS, Band 64, Nr. 7, 28 März 1966, Columbus, Ohio, USA J.BUECHI, P.FABIANI, H.U. FREY, A. HOFSTETTER, A. SCHORNO: "Synthesis and pharmacological properties of certain**

(73) Patentinhaber: **BAYER AG, D-5090 Leverkusen 1 Bayerwerk (DE)**

(72) Erfinder: **Sasse, Klaus, Dr., Pützweg 13, D-5060 Bergisch-Gladbach (DE)**
Erfinder: **Hänssler, Gerd, Dr., Am Arenzberg 58a, D-5090 Leverkusen 3 (DE)**
Erfinder: **Schmitt, Hans-Georg, Dr., Am Oberend 13, D-4150 Krefeld 1 (DE)**
Erfinder: **Paulus, Wilfried, Dr., Deswatinesstrasse 90, D-4150 Krefeld 1 (DE)**

(56) Entgegenhaltungen: (Fortsetzung)
**pyridylpyrazol-5-ones" Spalte 9 705, Zusammenfassung Nr. 9 705g**

**Die Akte enthält technische Angaben, die nach dem Eingang der Anmeldung eingereicht wurden und die nicht in dieser Patentschrift enthalten sind.**

EP 0 212 281 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue 1-Heteroaryl-4-aryl-pyrazol-Derivate, ein Verfahren zu ihrer Herstellung und ihre Verwendung als Mikrobizide.

Es ist bereits bekannt, daß bestimmte heterocyclische Verbindungen, wie z.B. N-Trichlormethylthio-phthalimid und -tetrahydrophthalimid, gute fungizide Wirkungen aufweisen (vgl. US-Patente 2 553 770, 2 553 771 und 2 553 776). Außerdem sind auch organische Schwefelverbindungen, wie beispielsweise das Zinkethylen-1,2-bis-(dithiocarbamat), fungizid gut wirksame Verbindungen (vgl. z.B. R. Wegler «Chemie der Pflanzenschutz- und Schädlingsbekämpfungsmittel», Springer Verlag, Berlin, Heidelberg, New York 1970, Band 2, S. 65 ff.). Die Wirkung dieser Verbindungen kann unter bestimmten Bedingungen, z.B. bei niedrigen Aufwandmengen und -konzentrationen, in einigen Anwendungsbereichen nicht immer völlig zufriedenstellend sein.

Weiterhin sind 1,4-Diaryl-pyrazolin-5-one bekannt, die als Herbizide eingesetzt werden. Über eine fungizide Wirksamkeit ist nichts bekannt (vgl. DE-OS 2 651 008).

Weiterhin sind 1-Heteroaryl-4-aryl-pyrazolin-5-one, wie z.B. 1-Pyrimidinyl-(2)-4-phenyl-pyrazolin-5-on sowie deren mikrobizide Eigenschaften bekannt (vgl. EP-A-165 448).

Es wurden neue 1-Heteroaryl-4-aryl-pyrazol-Derivate der Formeln (I) und (IA) gefunden,

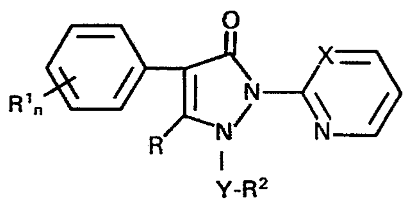

(I)

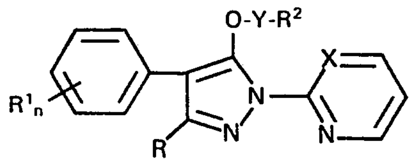

(IA)

in welchen

R für Wasserstoff oder Alkyl mit 1 bis 3 Kohlenstoffatomen steht,

$R^1$ für Fluor, Chlor, Brom, Hydroxy, Methyl, Ethyl, n-Propyl, iso-Propyl, tert.-Butyl, Trifluormethyl, Tetrachlorethyl, Dichlorfluormethyl, Alkoxy mit 1 bis 4 Kohlenstoffatomen, Trifluormethoxy, Dichlorfluormethoxy, Methoxymethoxy, Ethoxymethoxy, 2-Methoxyethoxy, 2-Ethoxyethoxy, Methylthiomethoxy, 2-Methylthioethoxy, 2-Ethylthioethoxy, Phenoxymethoxy, 2-Phenoxyethoxy, Alkylthio mit 1 bis 4 Kohlenstoffatomen, Trifluormethylthio, Dichlorfluormethylthio, Methoxymethylthio, 2-Methoxyethylthio, Ethoxymethylthio, 2-Ethoxyethylthio, Methylthiomethylthio, Amino, Dimethylamino, Diethylamino, Monomethylamino, Monoethylamino oder Acetylamino steht,

Y für CO oder $SO_2$ steht,

n für eine ganze Zahl 0, 1, 2 oder 3 steht,

$R^2$ für Wasserstoff, für gesättigtes Alkyl mit 1 bis 12 Kohlenstoffatomen, Halogenalkyl mit 1 bis 4 Kohlenstoffatomen und 1 bis 3 gleichen oder verschiedenen Fluor-, Brom- oder Chloratomen, für Hydroxyalkyl mit 1 bis 3 Kohlenstoffatomen, für Alkoxyalkyl und Alkylthioalkyl mit jeweils 1 bis 4 Kohlenstoffatomen in den Alkylresten, für Cycloalkyl mit 3 bis 7 Kohlenstoffatomen, das gegebenenfalls durch Methyl ein- bis vierfach substituiert sein kann, für gegebenenfalls ein- bis dreifach, gleich oder verschieden durch Alkyl mit 1 bis 4 Kohlenstoffatomen, Fluor, Chlor, Brom, Nitro, Trifluormethyl, Dichlorfluormethyl, Cyano, Methoxy, Ethoxy, Dimethylamino, Diethylamino, Acetylamino, Phenyl, Carbonyl, Carbonsäureamidgruppe oder Carbonsäureestergruppe substituiertes Phenyl steht, weiterhin für Furan, Tetrahydrofuran oder Benzofuran steht, die gegebenenfalls durch Methyl, Ethyl, Fluor, Chlor, Cyano, Phenyl, substituiertes Amino oder Carboxyl substituiert sein können für $OR^3$ auch für $SR^3$, wenn Y = CO ist, steht, worin $R^3$ für Alkyl mit 1 bis 6 Kohlenstoffatomen, Halogenalkyl mit 1 bis 3 Kohlenstoffatomen und 1 bis 5 gleichen oder verschiedenen Fluor- und Chloratomen, Phenyl steht, das gegebenenfalls ein- bis dreifach, gleich oder verschieden durch Methyl, Ethyl, Fluor, Chlor, Brom, $C_{1-4}$-Alkoxy, $C_{1-4}$-Dialkylamino, Nitro, Cyan oder Carbonsäureestergruppe substituiert ist,

$R^2$ für $-N\langle^{R^4}_{R^5}$ steht, worin $R^4$ für Wasserstoff oder Alkyl mit 1 bis 4 Kohlenstoffatomen und $R^5$ für Wasserstoff, Alkyl mit 1 bis 6 Kohlenstoffatomen stehen und

X für =CH- oder ein Stickstoffatom steht.

Weiterhin wurde gefunden, daß man die neuen 1-Heteroaryl-4-aryl-pyrazol-Derivate der Formeln (I) und (IA)

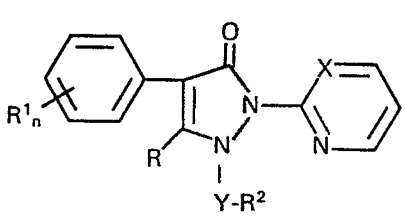

(I)

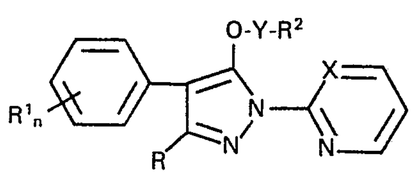

(IA)

in welchen

R für Wasserstoff oder Alkyl mit 1 bis 3 Kohlenstoffatomen steht,

$R^1$ für Fluor, Chlor, Brom, Hydroxy, Methyl, Ethyl, n-Propyl, iso-Propyl, tert.-Butyl, Trifluormethyl, Tetrachlorethyl, Dichlorfluormethyl, Alkoxy mit 1 bis 4 Kohlenstoffatomen, Trifluormethoxy, Dichlorfluormethoxy, Methoxymethoxy, Ethoxymethoxy, 2-Methoxyethoxy, 2-Ethoxyethoxy, Methylthiomethoxy, 2-Methylthioethoxy, 2-Ethylthioethoxy, Phenoxymethoxy, 2-Phenoxyethoxy, Alkylthio mit 1 bis 4 Kohlenstoffatomen, Trifluormethylthio, Dichlorfluormethylthio, Methoxymethylthio, 2-Methoxyethylthio, Ethoxymethylthio, 2-Ethoxyethylthio, Methylthiomethylthio, Amino, Dimethylamino, Diethylamino, Monomethylamino, Monoethylamino oder Acetylamino steht,

Y für CO oder $SO_2$ steht,

n für eine ganze Zahl 0, 1, 2 oder 3 steht,

$R^2$ für Wasserstoff, für gesättigtes Alkyl mit 1 bis 12 Kohlenstoffatomen, Halogenalkyl mit 1 bis 4 Kohlenstoffatomen und 1 bis 3 gleichen oder verschiedenen Fluor-, Brom- oder Chloratomen, für Hydroxyalkyl mit 1 bis 3 Kohlenstoffatomen, für Alkoxyalkyl und Alkylthioalkyl mit jeweils 1 bis 4 Kohlenstoffatomen in den Alkylresten, für Cycloalkyl mit 3 bis 7 Kohlenstoffatomen, das gegebenenfalls durch Methyl ein- bis vierfach substituiert sein kann, für gegebenenfalls ein- bis dreifach, gleich oder verschieden durch Alkyl mit 1 bis 4 Kohlenstoffatomen, Fluor, Chlor, Brom, Nitro, Trifluormethyl, Dichlorfluormethyl, Cyano, Methoxy, Ethoxy, Dimethylamino, Diethylamino, Acetylamino, Phenyl, Carbonyl, Carbonsäureamidgruppe oder Carbonsäureestergruppe substituiertes Phenyl steht, weiterhin für Furan, Tetrahydrofuran oder Benzofuran steht, die gegebenenfalls durch Methyl, Ethyl, Fluor, Chlor, Cyano, Phenyl, substituiertes Amino oder Carboxyl substituiert sein können für $OR^3$ auch für $SR^3$, wenn Y = CO ist, steht, worin $R^3$ für Alkyl mit 1 bis 6 Kohlenstoffatomen, Halogenalkyl mit 1 bis 3 Kohlenstoffatomen und 1 bis 5 gleichen oder verschiedenen Fluor- und Chloratomen, Phenyl steht, das gegebenenfalls ein- bis dreifach, gleich oder verschieden durch Methyl, Ethyl, Fluor, Chlor, Brom, $C_{1-4}$-Alkoxy, $C_{1-4}$-Dialkylamino, Nitro, Cyan oder Carbonsäureestergruppe substituiert ist,

$R^2$ für $-N\langle^{R^4}_{R^5}$ steht, worin $R^4$ für Wasserstoff oder Alkyl mit 1 bis 4 Kohlenstoffatomen und $R^5$ für Wasserstoff, Alkyl mit 1 bis 6 Kohlenstoffatomen stehen und

X für =CH- oder ein Stickstoffatom steht,

erhält wenn man 1-Heteroaryl-4-aryl-pyrazolin-5-one der Formeln (II) bzw. (IIA)

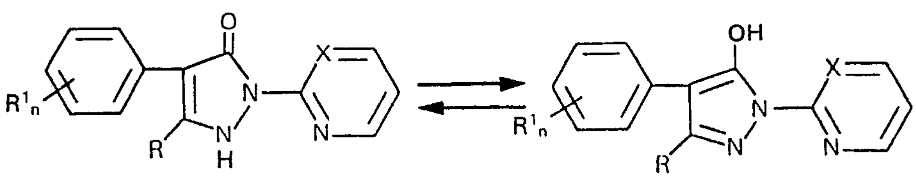

(II) (IIA)

in welchen

R, $R^1$, X und n die oben angegebenen Bedeutungen haben, mit einem reaktionsfähigen Derivat einer Säure der Formel (III)

$R^2$-Y-Z (III)

in welcher

$R^2$ und Y die oben angegebenen Bedeutungen besitzen und

Z für Halogen, einen Acyloxyrest, einen Sulfonyloxyrest oder einen Azolrest steht, gegebenenfalls in Gegenwart eines säurebindenden Mittels umsetzt.

Schließlich wurde gefunden, daß die 1-Heteroaryl-4-arylpyrazol-Derivate der Formeln (I) und (IA) zur Bekämpfung von mikrobiellen Parasiten eingesetzt werden können.

Dabei zeigen die erfindungsgemäßen Verbindungen der Formeln (I) und (IA) überraschenderweise eine höhere antimikrobielle Wirksamkeit als die aus dem Stand der Technik vorbekannten wirksamen Verbindungen, wie z.B. N-Trichlormethylthiophthalimid oder -tetrahydrophthalimid, Zinkethylen-1,2-bis-(dithiocarbamat) und/oder N,N-Dimethyl-N'-phenyl-N'-dichlorfluor-methylthio-sulfamid. Die erfindungsgemäßen Verbindungen stellen somit eine Bereicherung der Technik dar.

Die erfindungsgemässen 1-Heteroaryl-4-aryl-pyrazol-Derivate sind durch die Formel (I) bzw. (IA) allgemein definiert.

Bevorzugt sind Verbindungen der Formeln (I) und (IA), bei denen

R für Wasserstoff, Methyl oder Ethyl steht,

$R^1$ für Methoxy, Ethoxy, Trifluormethoxy, Methyl, Ethyl, Fluor, Chlor, Brom, Trifluormethyl, Methylthio, Ethylthio oder Trifluormethylthio steht,

n für eine ganze Zahl 0, 1 oder 2 steht,

X für =CH oder ein Stickstoffatom steht,

Y für CO oder $SO_2$ steht und

$R^2$ für Alkyl mit 1 bis 4 Kohlenstoffatomen, wie Methyl, Ethyl, n-Propyl, iso-Propyl, n-Butyl, iso-Butyl, sek.-Butyl und tert.-Butyl, für gegebenenfalls substituiertes Phenyl, Methoxy, Ethoxy, Phenoxy, Furfuryl, Dimethylamino oder Diethylamino steht.

Verwendet man beispielsweise 1-Pyrimidyl-(2)-4-phenyl-pyrazolin-5-on und Acetanhydrid als Ausgangsstoffe, so kann der Reaktionsablauf durch das folgende Formelschema wiedergegeben werden:

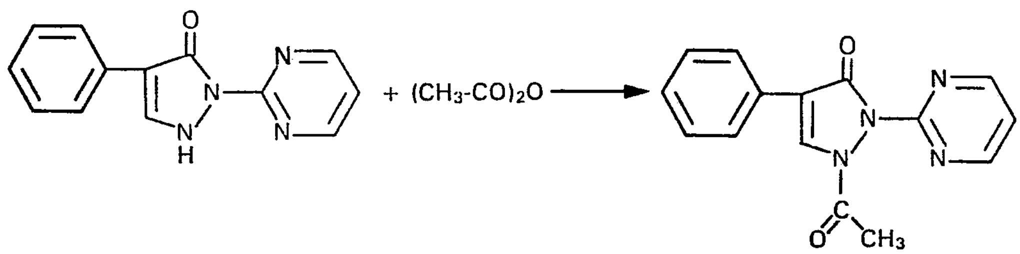


Verwendet man beispielsweise 1-Pyridyl-(2)-4-phenyl-pyrazolin-5-on, Furan-2-carbonsäurechlorid und Triethylamin als Ausgangsstoffe, so kann der Reaktionsablauf durch das folgende Formelschema wiedergegeben werden:

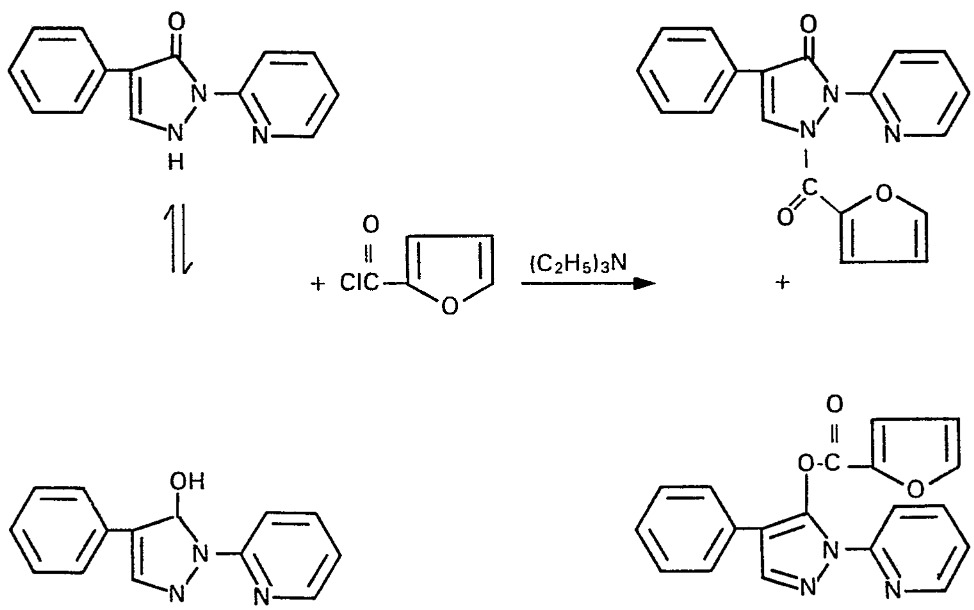


Verwendet man beispielsweise 1-Pyridyl-(2)-4-pyrazolin-5-on und Phenylisocyanat als Ausgangsstoffe, so kann der Reaktionsablauf durch das folgende Formelschema wiedergegeben werden:

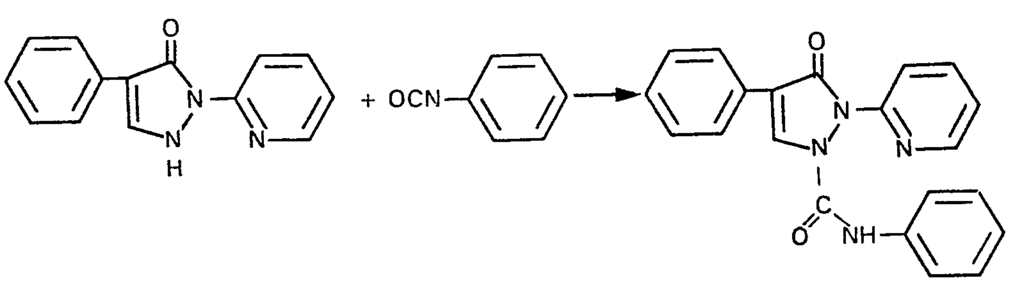

Die bei der Durchführung des erfindungsgemäßen Verfahrens als Ausgangsstoffe zu verwendenden 1-Pyri(mi)dyl-4-aryl-pyrazolin-5-one sind durch die Formel (II) bzw. (IIA) allgemein definiert. R, $R^1$, X und n haben darin die Bedeutung, die bereits im Zusammenhang mit der Beschreibung der erfindungsgemäßen Stoffe der Formel (I) für diese Symbole genannt wurde.

Die Ausgangsstoffe der Formel (II) bzw. (IIA) sind in der Literatur bisher nicht vorbeschrieben. Ihre Herstellung und Verwendung als Fungizide ist Gegenstand der Patentanmeldung EP-A-165 448. Man gewinnt sie gemäß Angaben in dieser Patentschrift durch Umsetzung von α-Acylphenylessigsäureestern oder deren Derivaten der Formel (IV) mit 2-Hydrazino-pyri(mi)din (V), die selbst jeweils literaturbekannte Verbindungen darstellen.

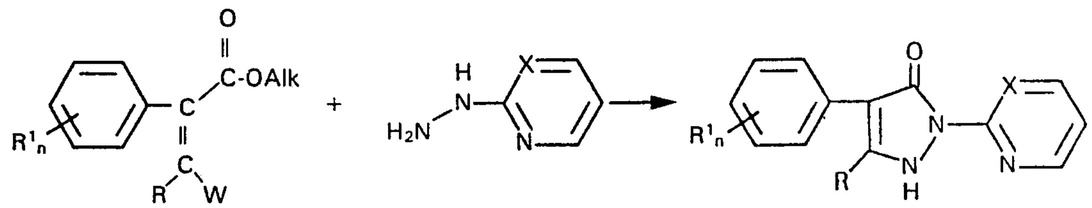

(IV) (V) (II)

worin

W für OH, OAlk, Hal oder $NAlk_2$ steht.

Als Beispiele für Ausgangsstoffe der Formel (II) seien genannt:

1-Pyridyl-(2)-4-phenyl-pyrazolin-5-on
1-Pyridyl-(2)-4-(4-fluor-phenyl)-pyrazolin-5-on
1-Pyridyl-(2)-4-(2-chlor-phenyl)-pyrazolin-5-on
1-Pyridyl-(2)-4-(2-brom-phenyl)-pyrazolin-5-on
1-Pyridyl-(2)-4-(3,4-dichlor-phenyl)-pyrazolin-5-on
1-Pyridyl-(2)-4-(2-methoxy-phenyl)-pyrazolin-5-on
1-Pyridyl-(2)-4-(4-methyl-phenyl)-pyrazolin-5-on
1-Pyridyl-(2)-4-(3-trifluormethoxy-phenyl)-pyrazolin-5-on
1-Pyridyl-(2)-4-(4-trifluormethylmercapto-phenyl)-pyrazolin-5-on
1-Pyridyl-(2)-4-(4-methyl-phenyl)-pyrazolin-5-on
1-Pyridyl-(2)-4-(3-trifluormethyl-phenyl)-pyrazolin-5-on
1-Pyridyl-(2)-3-methyl-4-phenyl-pyrazolin-5-on
1-Pyridyl-(2)-3-ethyl-4-phenyl-pyrazolin-5-on
1-Pyrimidyl-(2)-4-(2-fluor-phenyl)-pyrazolin-5-on
1-Pyrimidyl-(2)-4-(4-chlor-phenyl)-pyrazolin-5-on
1-Pyrimidyl-(2)-4-(2-fluor-6-chlor-phenyl)-pyrazolin-5-on
1-Pyrimidyl-(2)-4-(4-methoxy-phenyl)-pyrazolin-5-on
1-Pyrimidyl-(2)-4-(3,4-difluormethylendioxy-phenyl)-pyrazolin-5-on
1-Pyrimidyl-(2)-4-(2-methoxy-phenyl)-pyrazolin-5-on
1-Pyrimidyl-(2)-4-(2-chlor-5-trifluormethyl-phenyl)-pyrazolin-5-on
1-Pyrimidyl-(2)-3-methyl-4-phenyl-pyrazolin-5-on.

Die zur Herstellung der erfindungsgemäßen Verbindungen weiterhin als Ausgangsstoffe zu verwendenden Säurederivate der Formel (III) sind bekannt. Es handelt sich um die für Acylierungsreaktionen üblichen reaktiven Derivate der Carbonsäuren, der Kohlensäureester, Carbamidsäuren, Sulfonsäuren und Sulfamidsäuren, insbesondere Carbonsäurehalogenide, Carbonsäureanhydride, gemischte Anhydride aus Carbonsäuren und Kohlensäureestern, Carbonsäure-azolide, Ketene, Kohlensäureesterhalogenide, Dikohlensäureester, Carbamidsäurehalogenide, Isocyanate, Sulfonsäurehalogenide, Sulfonsäureanhydride und dergleichen.

Die Umsetzung der 1-Pyri(mi)dyl-4-aryl-pyrazolin-5-one der Formel (II) bzw. (IIA) mit den reaktiven Derivaten der Carbonsäuren, Kohlensäureestern, Carbamidsäuren, Sulfonsäuren oder Sulfamidsäuren der Formel (III) wird unter den für Acylierungsreaktionen grundsätzlich bekannten Bedingungen vorgenommen.

Werden Carbonsäureanhydride, gemischte Anhydride aus Carbonsäuren und Kohlensäureestern, Dikohlensäuredialkylester oder Sulfonsäureanhydride als Acylierungskomponente eingesetzt, kann dies ohne weitere Hilfsmittel geschehen, indem man die Reaktionskomponenten vorzugsweise im äquimolaren Verhältnis unverdünnt oder in Verdünnungsmitteln aufeinander einwirken läßt. Es kann aber auch zweckmäßig sein, das Acylierungsmittel im Überschuß bis zu einem weiteren Mol einzusetzen oder, wie im Falle von Carbonsäureanhydriden, dieses als Lösungsmittel in einem größeren Überschuß zu verwenden.

Zweckmäßigerweise nimmt man die Reaktionen in Verdünnungsmitteln vor, wobei alle gegenüber den Reaktionspartnern inerten Lösungsmittel verwendet werden können. Hierzu gehören Kohlenwasserstoffe, wie Benzin oder Toluol, Halogen-Kohlenwasserstoffe, wie Di-, Tri- und Tetrachlormethan, Ketone, wie Aceton oder Methyl-isopropyl-keton, Ether, wie Diethylether, Tetrahydrofuran oder Dioxan, ferner Dimethylsulfoxid, Tetrahydrothiophendioxid, Dimethylformamid.

Die Reaktionstemperaturen können in einem größeren Bereich variiert werden. Im allgemeinen arbeitet man zwischen −20°C und 150°C, vorzugsweise zwischen Raumtemperatur und dem Siedepunkt des verwendeten Lösungsmittels.

Werden Carbonsäurehalogenide, Kohlensäureesterhalogenide, Carbamidsäurehalogenide oder Sulfonsäurehalogenide als Acylierungskomponente eingesetzt, ist der Zusatz einer Hilfsbase zur Bindung der bei der Reaktion freiwerdenden Halogenwasserstoffe zweckmäßig. Als solche sind geeignet: Alkali- und Erdalkali-hydroxide und -carbonate sowie tertiäre Amine, wie z.B. Triethylamin, Pyridin und N,N-Dimethyl-anilin.

Gegenüber der Reaktionskomponente der Formel (II) bzw. (IIA) werden das Acylierungsmittel und die säurebildende Base vorzugsweise in jeweils äquimolaren Mengen eingesetzt, es kann jedoch zweckmäßig sein, entweder nur die Hilfsbase oder die Acylierungskomponente und die Hilfsbase im Überschuß bis zu einem weiteren Mol einzusetzen.

Eine Alternative in der Reaktionsführung besteht darin, daß man das Pyrazolinon der Formel (II) bzw. (IIA) in einem inerten Lösungsmittel zunächst in ein Metallsalz überführt und dieses dann mit dem Acylierungsmittel zur Reaktion bringt. Dieses kann z.B. mit starken Basen wie Natriumhydrid, Natrium- oder Kaliumamid oder Kalium-tert.-butylat geschehen.

Die Umsetzungen werden vorzugsweise in inerten

Lösungsmitteln ausgeführt, wobei die bereits oben genannten geeignet sind. Die Reaktionstemperaturen betragen −20°C bis 150°C, vorzugsweise 0 bis 100°C.

Werden Isocyanate als Acylierungskomponente eingesetzt, führt man die Reaktion in der Regel mit äquimolarer Menge der Reaktionskomponenten in den oben genannten Lösungsmitteln durch. Die Reaktionstemperaturen betragen 0 bis 100°C. Zur Beschleunigung der Reaktion können Aktivatoren, wie z.B. 1,4-Diazabicyclooctan (DABCO) und Zinn-II-octoat, verwendet werden.

Die Reaktionsprodukte werden in üblicher Weise isoliert durch Abdestillieren des Lösungsmittels, Auswaschen des gegebenenfals bei der Reaktion entstandenen Amin-hydrochlorids oder durch Ausfällung mit Wasser bei Durchführung der Reaktion in einem mit Wasser mischbaren Lösungsmittel.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind z.B. für den Gebrauch als Pflanzenschutzmittel, insbesondere als Fungizide, geeignet oder im Materialschutz zum Schutz technischer Materialien einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas oyringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Perono spora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck

gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Die erfindungsgemäßen Verbindungen der Formel (I) zeigen gute Wirkungen gegen Erreger von Getreidekrankheiten, gegen Erreger von Reiskrankheiten (Pyricularia oryzae), gegen Erreger von Gemüse- und Obstkrankheiten (Botrytis cinerea). Ebenfalls haben die Verbindungen bei entsprechender Anwendung auch eine bakterizide Wirkung, ebenso zeigen sie eine Wirkung im Plattentest.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylakoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthiobenzthiazol, Organo-Zinnverbindungen, Methylenbisthiocyanat, Phenolderivate, wie 2-Phenylphenol, (2,2'-Dihydroxy-5,5'-dichlor)diphenylmethan und 3-Methyl-4-chlor-phenol.

*Herstellungsbeispiele*

*Beispiel 1*

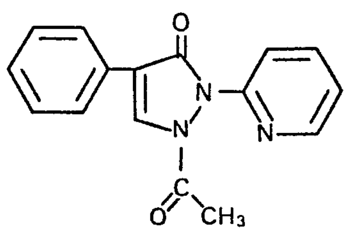

+

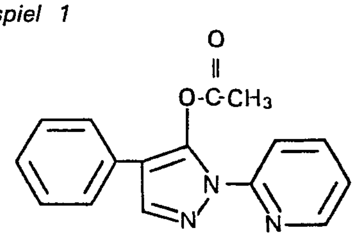

23,7 g (0,1 Mol) 1-Pyridyl-(2)-4-phenyl-pyrazolin-5-on werden in 150 ml Essigsäureanhydrid 6 Stunden unter Rückfluß gekocht. Überschüssiges Essigsäureanhydrid und gebildete Essigsäure werden im Vakuum abdestilliert. Der verbleibende Rückstand wird in 100 ml Essigsäureethylester gelöst, die Lösung mehrere Stunden in Eis gekühlt. Die abgeschiedenen Kristalle werden abgesaugt und getrocknet.

Man erhält 20,4 g (73% der Theorie) eines Gemisches aus 1-Pyridyl-(2)-2-acetyl-4-phenyl-pyrazolin-5-on und 1-Pyridyl-(2)-4-phenyl-5-acetoxypyrazol im Verhältnis 60 : 40 (nach $^1$H-Protonenresonanzspektrum) mit einem Schmelzpunkt: 158 - 162°C.

In analoger Weise erhält man:

*Beispiel 2*

1-Pyrimidyl-(2)-2-acetyl-4-phenyl-pyrazol-5-on. Schmelzpunkt: 171 - 173°C (aus Essigester).

*Beispiel 3*

1-Pyrimidyl-(2)-2-acetyl-3-methyl-4-phenyl-pyrazol-5-on. Schmelzpunkt: 138 - 140°C (aus Toluol).

Die Verbindungen nach Beispielen 2 und 3 sind laut $^1$H-Protonenresonanzspektrum einheitlich und enthalten laut $^{13}$C-Kernresonanzspektrum den Acetylrest in 2-Position.

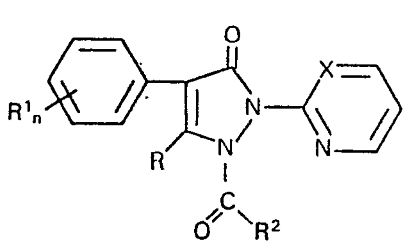

*Beispiel 4*

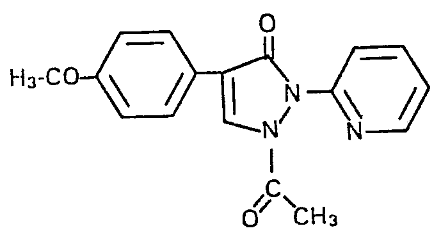

26,7g (0,1 Mol) 1-Pyridyl-(2)-4-(4-methoxy-phenyl)-pyrazolin-5-on werden in 200 ml Dioxan gelöst. Hierzu gibt man 10,1 g (0,1 Mol) Triethylamin und 0,2 g 4-Dimethylaminopyridin und tropft anschließend bei Raumtemperatur 7,9 g (0,1 Mol) Acetylchlorid ein. Die Mischung wird noch 1 Stunde bei Raumtemperatur und 3 Stunden bei 90°C nachgerührt, abgekühlt und in 1 l Wasser eingerührt. Die abgeschiedenen Kristalle werden abgesaugt, an der Luft getrocknet und aus Waschbenzin umkristallisiert. Man erhält 20,7 g (67% der Theorie) nur durch wenig 5-Acetoxy-derivat verunreinigtes 1-Pyridyl-(2)-2-acetyl-4-(4-methoxy-phenyl)-pyrazolin-5-on vom Schmelzpunkt 98 - 100°C.

In analoger Weise werden gewonnen:

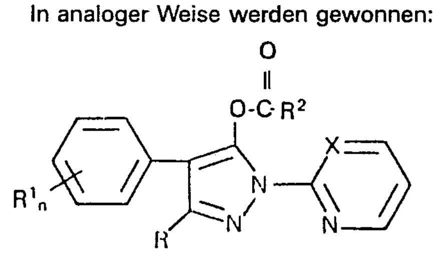

| *Beispiel* | X | $R^1$ | n | $R^2$ | R | F (°C) | Umkristallisiert aus |
|---|---|---|---|---|---|---|---|
| 5 a | N | — | O | $-C(CH_3)_3$ | H | 140-141 | Toluol |
| 6 a | CH | — | O | 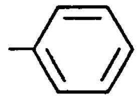 | H | 114-116 | Waschbenzin |
| 7 a | CH | $4-PCH_3$ | 1 | 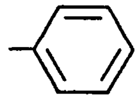 | H | 156-157 | Toluol |
| 8 a | CH | — | O | 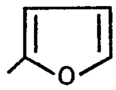 | $CH_3$ | 115-117 | Waschbenzin |
| 9 a | N | — | O | 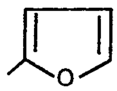 | H | 221-223 | Butanol |
| 9 b | N | — | O | 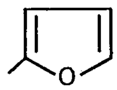 | H | 128-130 | Tetrachlormethan |
| 10 a | CH | — | O | $-O-CH_3$ | $CH_3$ | 150-152 | Waschbenzin |
| 11 a | N | — | O | $-O-CH_3$ | H | 174 | Toluol |
| 12 a | N | — | O | 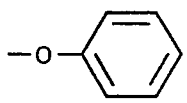 | H | 162-164 | Toluol/Ligroin |
| 13 a | N | $2-CH_3$ | 1 | 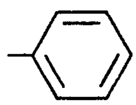 | H | 128,51 | Ethanol |
| 14 a | CH | $2-CH_3$ | 1 | 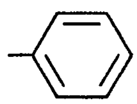 | H | 88-89 | Diisopropylether |
| 15 a | CH | $2-CH_3$ | 1 | 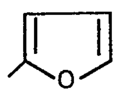 | H | 102-104 | Diisopropylether |
| 16 a | CH | 2-Cl, 6-F | 2 | 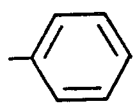 | H | 121-122 | Toluol |
| 17 a | N | 2-Cl, 6-F | 2 | 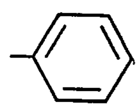 | H | 116-118 | Diisopropylether |
| 18 a | CH | — | O | 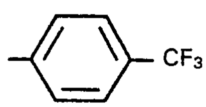 | H | 77-79 | Waschbenzin |

| *Beispiel* | X | $R^1$ | n | $R^2$ | R | F (°C) | Umkristallisiert aus |
|---|---|---|---|---|---|---|---|
| 19 a | CH | — | 0 | 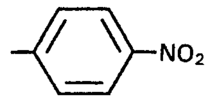 | H | 212-214 | Toluol |
| 20 a | CH | 3-Cl | 1 | 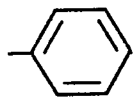 | H | 88-90 | Waschbenzin |
| 21 a | CH | 4-$CH_3$ | 1 | 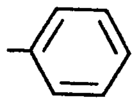 | H | 114-116 | Waschbenzin |
| 22 a | CH | 4-$CF_3$ | 1 | 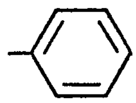 | H | 132-134 | Waschbenzin |
| 23 a | CH | 4-Cl | | 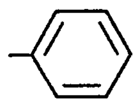 | H | 134-136 | Waschbenzin |
| 23 b | CH | 2-Cl, 6-F | 2 | 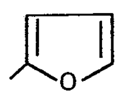 | H | 107-109 | Diisopropylether |
| 23 c | N | — | 0 | 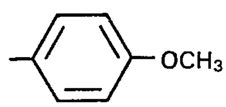 | H | 159-160 | Tetrachlorkohlenstoff |
| 23 d | N | 3-$CH_3$ | 1 | 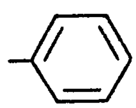 | H | 126-128 | Waschbenzin |
| 23 e | CH | 4-Cl | 1 | 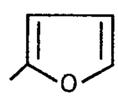 | H | 115-117 | Tetrachlorkohlenstoff |
| 23 f | N | 4-$OCH_3$ | 1 | 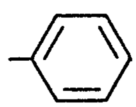 | H | 124-125 | Tetrachlorkohlenstoff |

*Beispiel 24*

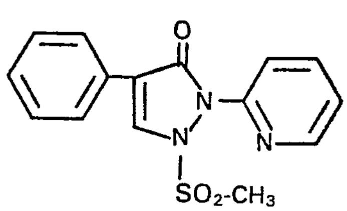

23,7 g (0,1 Mol) 1-Pyridyl-(2)-4-phenyl-pyrazolin-5-on werden in 200 ml Dioxan gelöst. Nach Zugabe von 10,1 g (0,1 Mol) Triethylamin tropft man bei Raumtemperatur unter schwacher Kühlung 11,5 g (0,1 Mol) Methansulfochlorid zu. Die Mischung wird noch 2 Stunden bei Raumtemperatur und 5 Stunden bei 70°C nachgerührt, abgekühlt und in 1 l Wasser eingerührt. Die Kristalle werden abgesaugt, an der Luft getrocknet und aus Ethanol umkristallisiert. Man erhält 22,5 g (71% d. Th.) 1-Pyridyl-(2)-2-methylsulfonyl-4-phenyl-pyrazolin-5-on vom Schmelzpunkt 112-114°C.

In analoger Weise gewinnt man:

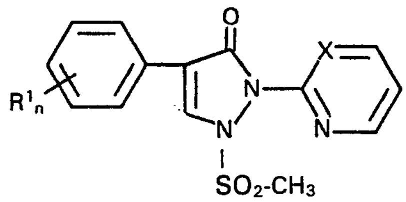

| Beispiel | X | R¹ | n | R² | F (°C) | Umkristallisiert aus |
|---|---|---|---|---|---|---|
| 25 | CH | — | 0 | 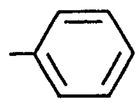 | 132-134 | Toluol |
| 26 | CH | 4-$OCH_3$ | 1 | 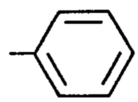 | 133-135 | Toluol |
| 27 | CH | — | 0 | $-N(CH_3)_2$ | 135-137 | Toluol |
| 28 | N | — | 0 | $-N(CH_3)_2$ | 166-168 | Toluol |

Tabelle (Fortsetzung)

| Beispiel | X | R¹ | n | R² | R | F (°C) | Umkristallisation |
|---|---|---|---|---|---|---|---|
| 29 | CH | 2-Cl | 1 | 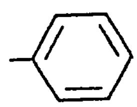 | H | 135 | Ethanol |
| 30 | CH | 2-Cl, 6-F | 2 | 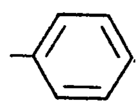 | H | 128-130 | Diisopropylether |
| 31 | N | 2-Cl, 6-F | 2 | 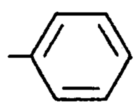 | H | 198-200 | Butanol |
| 32 | CH | 2-$CH_3$ | 1 | 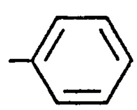 | H | 191-121 | Waschbenzin |
| 33 | CH | 3-Cl | 1 | 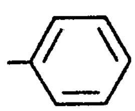 | H | 124-126 | Waschbenzin |
| 34 | CH | 4-$CH_3$ | 1 | 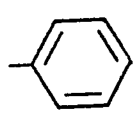 | H | 132-134 | Toluol |

Tabelle (Fortsetzung)

| Beispiel | X | $R^1$ | n | $R^2$ | R | F (°C) | Umkristallisation |
|---|---|---|---|---|---|---|---|
| 35 | CH | 4-$CF_3$ | 1 | 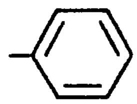 | H | 159-161 | Toluol |
| 36 | CH | 4-Cl | 1 | 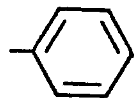 | H | 138-140 | Butanol |

*Anwendungsbeispiele*

In den folgenden Anwendungsbeispielen wird die nachfolgend aufgeführte Verbindung als Vergleichssubstanz eingesetzt:

$CH_2$-NH-CS-S
Zn (A)
$CH_2$-NH-CS-S

Zinkethylen-1,2-bis-(dithiocarbamat)

*Beispiel A*

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäss folgender Herstellungsbeispiele: 1, 2, 9a und 24.

*Beispiel B*

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2wöchiger Lagerung bei 28°C und 60 bis 70% rel. Luftfeuchtigkeit wird die MHK bestimmt. MHK ist die niedrigste Konzentration an Wirkstoff, bei der keinerlei Bewuchs durch die verwendete Mikrobenart erfolgt. Gute Wirkungen zeigen z.B. die Verbindungen entsprechend den Herstellungsbeispielen 2, 6a, 12a, 9b, 24 und 25 an den Testorganismen:

Alternaria tenuis
Aspergillus niger
Aureobasidium pullulans
Chaetomium globosum
Cladosporium cladosporioides
Lentinus tigrinus
Penicillium glaucum
Polyporus versicolor
Sclerophoma pityophila

*Beispiel C*

Wirkung gegen Bakterien

Ein Agar, der als Nährmedium Bouillon enthält, wird mit erfindungsgemäßen Wirkstoffen in Konzentrationen von 1 bis 5000 ppm versetzt. Darauf infiziert man das Nährmedium jeweils mit den in Tabelle C aufgeführten Testorganismen und hält das infizierte Medium 2 Wochen bei 28°C und 60 bis 70% rel. Luftfeuchtigkeit. Die MHK ist die niedrigste Konzentration an Wirkstoff, bei der keinerlei Bewuchs durch die verwendete Mikrobenart erfolgt. Die Verbindungen gemäß den Herstellungsbeispielen 2, 6a, 12a, 9b, 13 und 14 zeigen gute Wirkungen.

Tabelle C

Angabe der MHK-Werte in mg/l bei der Einwirkung der unten angegebenen Wirkstoffe auf Bakterien.

| Testorganismen | MHK in mg/l der Wirkstoffe Beispiel Nr. 2 | 6a | 12a | 9b | 24 | 25 |
|---|---|---|---|---|---|---|
| Escherichia coli | 750 | 350 | 200 | 50 | 1000 | |
| Staphylococcus aureus | 1000 | 100 | 500 | 500 | 50 | 20 |

*Beispiel D*

Wirkung gegen Schleimorganismen

Erfindungsgemäße Substanzen werden in Konzentrationen von jeweils 0,1 bis 100 mg/l in Allens

Nährlösung [Arch. Mikrobiol. 17, 34 bis 53 (1952)], die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Eine gute Wirkung zeigt die Verbindung gemäß Herstellungsbeispiel 13.

Tabelle D

MHK-Wert in mg/l bei der Einwirkung der unten angegebenen Substanz auf Schleimorganismen

| Wirkstoff | MHK in mg/l |
|---|---|
| Beispiel Nr. 24 | 2 - < 5 |

**Patentansprüche**

1. 1-Heteroaryl-4-phenyl-pyrazol-Derivate der Formeln (I) und (IA),

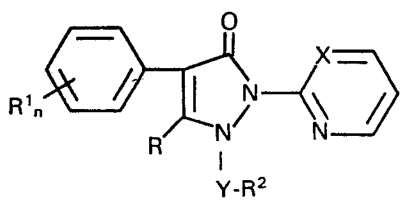

(I)

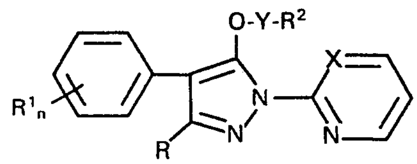

(IA)

in welchen

R für Wasserstoff oder Alkyl mit 1 bis 3 Kohlenstoffatomen steht,

$R^1$ für Fluor, Chlor, Brom, Hydroxy, Methyl, Ethyl, n-Propyl, iso-Propyl, tert.-Butyl, Trifluormethyl, Tetrachlorethyl, Dichlorfluormethyl, Alkoxy mit 1 bis 4 Kohlenstoffatomen, Trifluormethoxy, Dichlorfluormethoxy, Methoxymethoxy, Ethoxymethoxy, 2-Methoxyethoxy, 2-Ethoxyethoxy, Methylthiomethoxy, 2-Methylthioethoxy, 2-Ethylthioethoxy, Phenoxymethoxy, 2-Phenoxyethoxy, Alkylthio mit 1 bis 4 Kohlenstoffatomen, Trifluormethylthio, Dichlorfluormethylthio, Methoxymethylthio, 2-Methoxyethylthio, Ethoxymethylthio, 2-Ethoxyethylthio, Methylthiomethylthio, Amino, Dimethylamino, Diethylamino, Monomethylamino, Monoethylamino oder Acetylamino steht,

Y für CO oder $SO_2$ steht,

n für eine ganze Zahl 0, 1, 2 oder 3 steht,

$R^2$ für Wasserstoff, für gesättigtes Alkyl mit 1 bis 12 Kohlenstoffatomen, Halogenalkyl mit 1 bis 4 Kohlenstoffatomen und 1 bis 3 gleichen oder verschiedenen Fluor-, Brom- oder Chloratomen, für Hydroxyalkyl mit 1 bis 3 Kohlenstoffatomen, für Alkoxyalkyl und Alkylthioalkyl mit jeweils 1 bis 4 Kohlenstoffatomen in den Alkylresten, für Cycloalkyl mit 3 bis 7 Kohlenstoffatomen, das gegebenenfalls durch Methyl ein- bis vierfach substituiert sein kann, für gegebenenfalls ein- bis dreifach, gleich oder verschieden durch Alkyl mit 1 bis 4 Kohlenstoffatomen, Fluor, Chlor, Brom, Nitro, Trifluormethyl, Dichlorfluormethyl, Cyano, Methoxy, Ethoxy, Dimethylamino, Diethylamino, Acetylamino, Phenyl, Carbonyl, Carbonsäureamidgruppe oder Carbonsäureestergruppe substiuiertes Phenyl steht, weiterhin für Furan, Tetrahydrofuran oder Benzofuran steht, die gegebenenfalls durch Methyl, Ethyl, Fluor, Chlor, Cyano, Phenyl, substituiertes Amino oder Carboxyl substituiert sein können für $OR^3$ auch für $SR^3$, wenn Y = CO ist, steht, worin $R^3$ für Alkyl mit 1 bis 6 Kohlenstoffatomen, Halogenalkyl mit 1 bis 3 Kohlenstoffatomen und 1 bis 5 gleichen oder verschiedenen Fluor- und Chloratomen, Phenyl steht, das gegebenenfalls ein- bis dreifach, gleich oder verschieden durch Methyl, Ethyl, Fluor, Chlor, Brom, $C_{1-4}$-Alkoxy, $C_{1-4}$-Dialkylamino, Nitro, Cyan oder Carbonsäureestergruppe substituiert ist,

$R^2$ für $-N<^{R^4}_{R^5}$ steht, worin $R^4$ für Wasserstoff oder Alkyl mit 1 bis 4 Kohlenstoffatomen und $R^5$ für Wasserstoff, Alkyl mit 1 bis 6 Kohlenstoffatomen stehen und

X für =CH- oder ein Stickstoffatom steht.

2. 1-Heteroaryl-4-aryl-pyrazol-Derivate der Formeln (I) und (IA) gemäß Anspruch 1, worin

R für Wasserstoff, Methyl oder Ethyl steht,

$R^1$ für Methoxy, Ethoxy, Trifluormethoxy, Methyl, Ethyl, Fluor, Chlor, Brom, Trifluormethyl, Methylthio, Ethylthio oder Trifluormethylthio steht,

n für eine ganze Zahl 0, 1 oder 2 steht,

X für =CH- oder ein Stickstoffatom steht,

Y für CO oder $SO_2$ steht und

$R^2$ für Alkyl mit 1 bis 4 Kohlenstoffatomen, für gegebenenfalls substituiertes Phenyl, Methoxy, Ethoxy, Phenoxy, Furfuryl, Dimethylamino oder Diethylamino steht.

3. Verfahren zur Herstellung von 1-Heteroaryl-4-aryl-pyrazol-Derivaten der Formeln (I) und (IA)

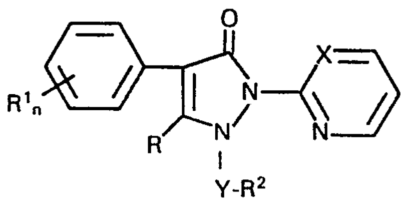

(I)

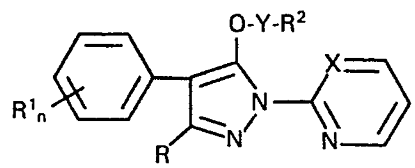

(IA)

in welcher

R für Wasserstoff oder Alkyl mit 1 bis 3 Kohlenstoffatomen steht,

$R^1$ für Fluor, Chlor, Brom, Hydroxy, Methyl, Ethyl, n-Propyl, iso-Propyl, tert.-Butyl, Trifluormethyl, Tetrachlorethyl, Dichlorfluormethyl, Alkoxy mit 1 bis 4 Kohlenstoffatomen, Trifluormethoxy, Dichlorfluormethoxy, Methoxymethoxy, Ethoxymethoxy, 2-Methoxyethoxy, 2-Ethoxyethoxy, Methylthiomethoxy, 2-Methylthioethoxy, 2-Ethylthioethoxy, Phenoxymethoxy, 2-Phenoxyethoxy, Alkylthio mit 1 bis 4 Kohlenstoffatomen, Trifluormethylthio, Dichlorfluormethylthio, Methoxymethylthio, 2-Methoxyethylthio, Ethoxymethylthio, 2-Ethoxyethylthio, Methylthiomethylthio, Amino, Dimethylamino, Diethylamino, Monomethylamino, Monoethylamino oder Acetylamino steht,

Y für CO oder $SO_2$ steht,

n für eine ganze Zahl 0, 1, 2 oder 3 steht,

$R^2$ für Wasserstoff, für gesättigtes Alkyl mit 1 bis 12 Kohlenstoffatomen, Halogenalkyl mit 1 bis 4 Kohlenstoffatomen und 1 bis 3 gleichen oder verschiedenen Fluor-, Brom- oder Chloratomen, für Hydroxyalkyl mit 1 bis 3 Kohlenstoffatomen, für Alkoxyalkyl und Alkylthioalkyl mit jeweils 1 bis 4 Kohlenstoffatomen in den Alkylresten, für Cycloalkyl mit 3 bis 7 Kohlenstoffatomen, das gegebenenfalls durch Methyl ein- bis vierfach substituiert sein kann, für gegebenenfalls ein- bis dreifach, gleich oder verschieden durch Alkyl mit 1 bis 4 Kohlenstoffatomen, Fluor, Chlor, Brom, Nitro, Trifluormethyl, Dichlorfluormethyl, Cyano, Methoxy, Ethoxy, Dimethylamino, Diethylamino, Acetylamino, Phenyl, Carbonyl, Carbonsäureamidgruppe oder Carbonsäureestergruppe substiuiertes Phenyl steht, weiterhin für Furan, Tetrahydrofuran oder Benzofuran steht, die gegebenenfalls durch Methyl, Ethyl, Fluor, Chlor, Cyano, Phenyl, substituiertes Amino oder Carboxyl substituiert sein können für $OR^3$ oder auch für $SR^3$, wenn Y = CO ist, steht, worin $R^3$ für Alkyl mit 1 bis 6 Kohlenstoffatomen, Halogenalkyl mit 1 bis 3 Kohlenstoffatomen und 1 bis 5 gleichen oder verschiedenen Fluor- und Chloratomen oder Phenyl steht, das gegebenenfalls ein- bis dreifach, gleich oder verschieden durch Methyl, Ethyl, Fluor, Chlor, Brom, $C_{1-4}$-Alkoxy, $C_{1-4}$-Dialkylamino, Nitro, Cyan oder Carbonsäureestergruppe substituiert ist,

$R^2$ für $-N\begin{smallmatrix}R^4\\R^5\end{smallmatrix}$ steht, worin $R^4$ für Wasserstoff oder Alkyl mit 1 bis 4 Kohlenstoffatomen und $R^5$ für Wasserstoff, Alkyl mit 1 bis 6 Kohlenstoffatomen stehen und

X für =CH- oder ein Stickstoffatom steht, dadurch gekennzeichnet, daß man 1-Heteroaryl-4-aryl-pyrazolin-5-one der Formeln (II) bzw. (IIA)

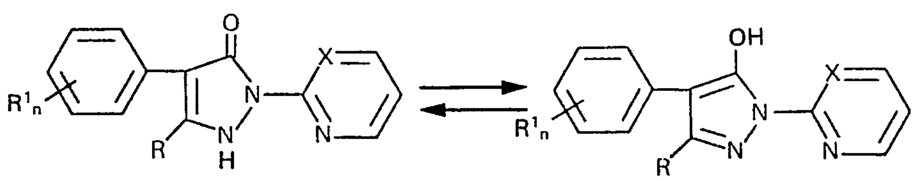

(II) (IIA)

in welchen

R, $R^1$, X und n die oben angegebenen Bedeutungen haben, mit einem reaktionsfähigen Derivat einer Säure der Formel (III)

$$R^2\text{-}Y\text{-}Z \qquad \text{(III)}$$

in welcher

$R^2$ und Y die oben angegebenen Bedeutungen besitzen und

Z für Halogen, einen Acyloxyrest, einen Sulfonyloxyrest oder einen Azolrest steht, gegebenenfalls in Gegenwart eines säurebindenden Mittels umsetzt.

4. Mikrobizide Mittel, gekennzeichnet durch einen Gehalt an mindestens einem 1-Heteroaryl-4-aryl-pyrazol-Derivat der Formeln (I) bzw. (IA) gemäß den Ansprüchen 1 bis 3.

5. Verfahren zur Bekämpfung von Mikroben, dadurch gekennzeichnet, daß man 1-Heteroaryl-4-aryl-pyrazol-Derivate der Formeln (I) bzw. (IA) gemäß den Ansprüchen 1 bis 3 auf Mikroben und/oder ihren Lebensraum einwirken läßt.

6. Verwendung von 1-Heteroaryl-4-aryl-pyrazol-Derivaten der Formel (I) bzw. (IA) gemäß den Ansprüchen 1 bis 3 zur Bekämpfung von Mikroben.

7. Verfahren zur Herstellung von mikrobiziden

Mitteln, dadurch gekennzeichnet, daß man 1-Heteroaryl-4-aryl-pyrazol-Derivate der Formeln (I) bzw. (IA) gemäß den Ansprüchen 1 bis 3 mit Streckmitteln und/oder oberflächenaktiven Mitteln vermischt.

**Claims**

1. Heteroaryl-4-phenyl-pyrazole derivatives of the formulae (I) and (IA)

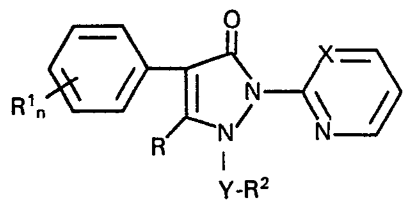

(I)

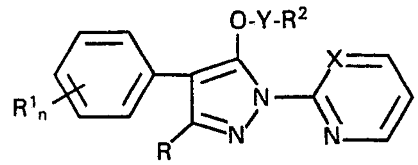

(IA)

in which

R represents hydrogen or alkyl with 1 to 3 carbon atoms,

$R^1$ represents fluorine, chlorine, bromine, hydroxyl, methyl, ethyl, n-propyl, iso-propyl, tert.-butyl, trifluoromethyl, tetrachloroethyl, dichlorofluoromethyl, alkoxy with 1 to 4 carbon atoms, trifluoromethoxy, dichlorofluoromethoxy, methoxymethoxy, ethoxymethoxy, 2-methoxyethoxy, 2-ethoxyethoxy, methylthiomethoxy, 2-methylthioethoxy, 2-ethylthioethoxy, phenoxymethoxy, 2-phenoxyethoxy, alkylthio with 1 to 4 carbon atoms, trifluoromethylthio, dichlorofluoromethylthio, methoxymethylthio, 2-methoxyethylthio, ethoxymethylthio, 2-ethoxyethylthio, methylthiomethylthio, amino, dimethylamino, diethylamino, monomethylamino, monoethylamino or acetylamino,

Y represents CO or $SO_2$,

n represents an integer 0, 1, 2 or 3,

$R^2$ represents hydrogen, or represents saturated alkyl with 1 to 12 carbon atoms, halogenoalkyl with 1 to 4 carbon atoms and 1 to 3 identical or different fluorine, bromine or chlorine atoms, or represents hydroxyalkyl with 1 to 3 carbon atoms, or represents alkoxyalkyl or alkylthioalkyl with in each case 1 to 4 carbon atoms in the alkyl radicals, or represents cycloalkyl which has 3 to 7 carbon atoms and can optionally be mono-, di-, tri- or tetrasubstituted by methyl, or represents phenyl which is optionally mono-, di- or trisubstituted by identical or different substituents from the group comprising alkyl with 1 to 4 carbon atoms, fluorine, chlorine, bromine, nitro, trifluoromethyl, dichlorofluoromethyl, cyano, methoxy, ethoxy, dimethylamino, diethylamino, acetylamino, phenyl, carbonyl, a carboxylic acid ester group, a carboxylic acid amide, or furthermore represents furan, tetrahydrofuran or benzofuran, which can optionally be substituted by methyl, ethyl, fluorine, chlorine cyano, phenyl, substituted amino or carbonyl, or represents $OR^3$ or also represents $SR^3$, if Y = CO, wherein $R^3$ represents alkyl with 1 to 6 carbon atoms, halogenoalkyl with 1 to 3 carbon atoms and 1 to 5 identical or different fluorine and chlorine atoms, or phenyl, which is optionally mono-, di- or trisubstituted by identical or different substituents from the group comprising methyl, ethyl, fluorine, chlorine, bromine, $C_{1-4}$-alkoxy, $C_{1-4}$-dialkylamino, nitro, cyano or a carboxylic acid ester group, or

$R^2$ represents $-N{<}^{R^4}_{R^5}$ wherein $R^4$ represents hydrogen or alkyl with 1 to 4 carbon atoms and $R^5$ represents hydrogen or alkyl with 1 to 6 carbon atoms, and

X represents = CH- or a nitrogen atom.

2. 1-Heteroaryl-4-aryl-pyrazole derivatives of the formulae (I) and (IA) according to claim 1, wherein

R represents hydrogen, methyl or ethyl,

$R^1$ represents methoxy, ethoxy, trifluoromethoxy, methyl, ethyl, fluorine, chlorine, bromine, trifluoromethyl, methylthio, ethylthio, trifluoromethylthio,

n represents an integer 0, 1 or 2,

X represents =CH- or a nitrogen atom,

Y represents CO or $SO_2$ and

$R^2$ represents alkyl with 1 to 4 carbon atoms, or represents optionally substituted phenyl, methoxy, ethoxy, phenoxy, furfuryl, dimethylamino or diethylamino.

3. Process for the preparation of 1-heteroaryl-4-aryl-pyrazole derivatives of the formulae (I) and (IA)

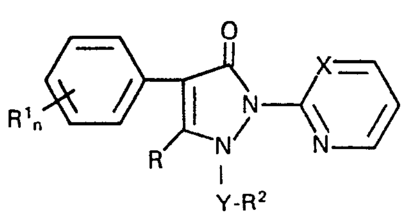

(I)

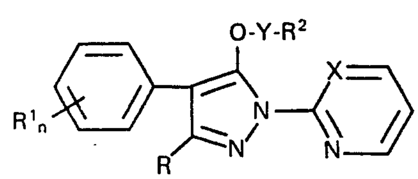

(IA)

in which
R represents hydrogen or alkyl with 1 to 3 carbon atoms,
$R^1$ represents fluorine, chlorine, bromine, hydroxyl, methyl, ethyl, n-propyl, iso-propyl, tert.-butyl, trifluoromethyl, tetrachloroethyl, dichlorofluoromethyl, alkoxy with 1 to 4 carbon atoms, trifluoromethoxy, dichlorofluoromethoxy, methoxymethoxy, ethoxymethoxy, 2-methoxyethoxy, 2-ethoxyethoxy, methylthiomethoxy, 2-methylthioethoxy, 2-ethylthioethoxy, phenoxymethoxy, 2-phenoxyethoxy, alkylthio with 1 to 4 carbon atoms, trifluoromethylthio, dichlorofluoromethylthio, methoxymethylthio, 2-methoxyethylthio, ethoxymethylthio, 2-ethoxyethylthio, methylthiomethylthio, amino, dimethylamino, diethylamino, monomethylamino, monoethylamino or acetylamino,
Y represents CO or $SO_2$,
n represents an integer 0, 1, 2 or 3,
$R^2$ represents hydrogen, or represents saturated alkyl with 1 to 12 carbon atoms, halogenoalkyl with 1 to 4 carbon atoms and 1 to 3 identical or different fluorine, bromine or chlorine atoms, or represents hydroxyalkyl with 1 to 3 carbon atoms, or represents alkoxyalkyl or alkylthioalkyl with in each case 1 to 4 carbon atoms in the alkyl radicals, or represents cycloalkyl which has 3 to 7 carbon atoms and can optionally be mono-, di-, tri- or tetrasubstituted by methyl, or represents phenyl which is optionally mono-, di- or trisubstituted by identical or different substituents from the group comprising alkyl with 1 to 4 carbon atoms, fluorine, chlorine, bromine, nitro, trifluoromethyl, dichlorofluoromethyl, cyano, methoxy, ethoxy, dimethylamino, diethylamino, acetylamino, phenyl, carbonyl, a carboxylic acid amide group, a carboxylic acid ester group, or furthermore represents furan, tetrahydrofuran or benzofuran, which can optionally be substituted by methyl, ethyl, fluorine, chlorine cyano, phenyl, substituted amino or carboxyl, or represents $OR^3$ or also represents $SR^3$, if Y = CO, wherein $R^3$ represents alkyl with 1 to 6 carbon atoms, halogenoalkyl with 1 to 3 carbon atoms and 1 to 5 identical or different fluorine and chlorine atoms, or phenyl, which is optionally mono-, di- or trisubstituted by identical or different substituents from the group comprising methyl, ethyl, fluorine, chlorine, bromine, $C_{1-4}$-alkoxy, $C_{1-4}$-dialkylamino, nitro, cyano or a carboxylic acid ester group, or
$R^2$ represents $-N{<}^{R^4}_{R^5}$ wherein $R^4$ represents hydrogen or alkyl with 1 to 4 carbon atoms and $R^5$ represents hydrogen or alkyl with 1 to 6 carbon atoms, and
X represents = CH- or a nitrogen atom,
characterized in that 1-heteroaryl-4-aryl-pyrazolin-5-ones of the formula (II) and (IIA)

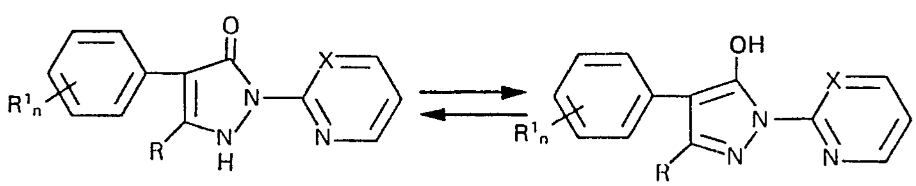

(II) (IIA)

in which
R, $R^1$, X and n have the abovementioned meanings, are reacted with a reactive derivative of an acid of the formula (III)

$R^2$-Y-Z (III)

in which
$R^2$ and Y have the abovementioned meanings and
Z represents halogen, an acyloxy radical, a sulphonyloxy radical or an azole radical, if appropriate in the presence of an acid-binding agent.

4. Microbicidal agents, characterized in that they contain at least one 1-heteroaryl-4-aryl-pyrazole derivative of the formula (I) and (IA) according to claims 1 to 3.

5. Method of combating microbes, characterized in that 1-heteroaryl-4-aryl-pyrazole derivatives of the formulae (I) and (IA) according to claims 1 to 3 are allowed to act on microbes and/or their environment.

6. Use of 1-heteroaryl-4-aryl-pyrazole derivatives of the formulae (I) and (IA) according to claims 1 to 3 for combating microbes.

7. Process for the preparation of microbicidal agents, characterized in that 1-heteroaryl-4-aryl-pyrazole derivatives of the formulae (I) and (IA) according to claims 1 to 3 are mixed with extenders and/or surface-active agents.

**Revendications**

1. Dérivés de 1-hétéroaryl-4-phényl-pyrazoles de formules I et IA

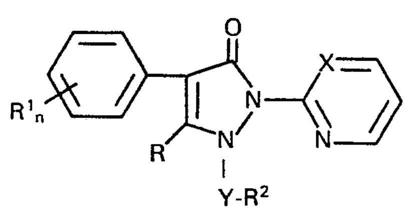

(I)

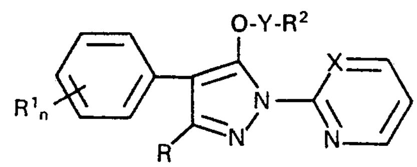

(IA)

dans lesquelles

R représente l'hydrogène ou un groupe alkyle en $C_1$-$C_3$,

$R^1$ représente le fluor, le chlore, le brome, un groupe hydroxy, méthyle, éthyle, n-propyle, isopropyle, tert.-butyle, trifluorométhyle, tétrachloroéthyle, dichlorofluorométhyle, alcoxy en $C_1$-$C_4$, trifluorométhoxy, dichlorofluorométhoxy, méthoxyméthoxy, éthoxyméthoxy, 2-méthoxyéthoxy, 2-éthoxyéthoxy, méthylthiométhoxy, 2-méthylthioéthoxy, 2-éthylthioéthoxy, phénoxyméthoxy, 2-phénoxyéthoxy, alkylthio en $C_1$-$C_4$, trifluorométhylthio, dichlorofluorométhylthio, méthoxyméthylthio, 2-méthoxyéthylthio, éthoxyméthylthio, 2-éthoxyéthylthio, méthylthiométhylthio, amino, diméthylamino, diéthylamino, monométhylamino, monoéthylamino ou acétylamino,

Y représente CO ou $SO_2$,

n est un nombre entier égal à 0, 1, 2 ou 3,

$R^2$ représente l'hydrogène, un groupe alkyle saturé en $C_1$-$C_{12}$, un groupe halogénoalkyle contenant 1 à 4 atomes de carbone et 1 à 3 atomes identiques ou différents de fluor, de brome ou de chlore, un groupe hydroxyalkyle en $C_1$-$C_3$ alkoxyalkyle ou alkylthioalkyle contenant chacun 1 à 4 atomes de carbone dans les groupes alkyle, un groupe cycloalkyle en $C_3$-$C_7$ qui peut éventuellement porter 1 à 4 substituants méthyle, un groupe phényle portant éventuellement 1 à 3 substituants identiques ou différents choisis parmi les groupes alkyle en $C_1$-$C_4$, le fluor, le chlore, le brome, les groupes nitro, trifluorométhyle, dichlorofluorométhyle, cyano, méthoxy, éthoxy, diméthylamino, diéthylamino, acétylamino, phényle, carbonyle, carboxamide ou ester d'acides carboxylique, ou encore le furanne, le tétrahydrofuranne ou le benzofuranne, qui peuvent éventuellement être substitués par des groupes méthyle, éthyle, le fluor, le chlore, des groupes cyano, phényle, amino substitué ou carboxyle, ou un groupe $OR^3$ ou encore $SR^3$ lorsque Y = CO, $R^3$ représentant un groupe alkyle en $C_1$-$C_6$, halogénoalkyle contenant 1 à 3 atomes de carbone et 1 à 5 atomes identiques ou différents de fluor ou de chlore, un groupe phényle portant éventuellement 1 à 3 substituants identiques ou différents choisi parmi les groupes méthyle, éthyle, le fluor, le chlore, le brome, les groupes alcoxy en $C_1$-$C_4$, di-(alkyle en $C_1$-$C_4$)-amino, nitro, cyano ou ester d'acide carboxylique,

$R^2$ représente $-N\langle^{R^4}_{R^5}$ dans lequel $R^4$ représente l'hydrogène ou un groupe alkyle en $C_1$-$C_4$ et $R^5$ représente l'hydrogène, un groupe alkyle en $C_1$-$C_6$, et

X représente =CH- ou un atome d'azote.

2. Dérivés de 1-hétéroaryl-4-aryl-pyrazoles de formules I et IA selon la revendication 1, dans lesquelles

R représente l'hydrogène, un groupe méthyle ou éthyle,

$R^1$ représente un groupe méthoxy, éthoxy, trifluorométhoxy, méthyle, éthyle, le fluor, le chlore, le brome, un groupe trifluorométhyle, méthylthio, éthylthio ou trifluorométhylthio,

n est un nombre entier égal à 0, 1 ou 2,

X représente =CH- ou un atome d'azote,

Y représente CO ou $SO_2$, et

$R^2$ représente un groupe alkyle, en $C_1$-$C_4$, un groupe phényle éventuellement substitué, un groupe méthoxy, éthoxy, phénoxy, furfuryle, diméthylamino ou diéthylamino.

3. Procédé de préparation des dérivés de 1-hétéroaryl-4-aryl-pyrazoles de formules I et IA

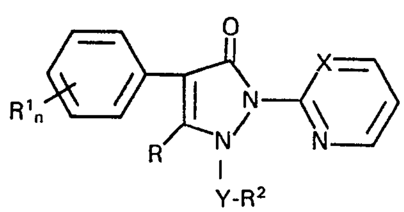

(I)

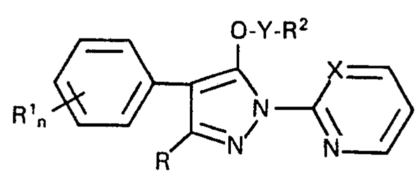

(IA)

dans lesquelles

R représente l'hydrogène ou un groupe alkyle en $C_1$-$C_3$,

$R^1$ représente le fluor, le chlore, le brome, un groupe hydroxy, méthyle, éthyle, n-propyle, isopropyle, tert.-butyle, trifluorométhyle, tétrachloroéthyle, dichlorofluorométhyle, alcoxy en $C_1$-$C_4$, trifluorométhoxy, dichlorofluorométhoxy, méthoxyméthoxy, éthoxyméthoxy, 2-méthoxyéthoxy, 2-éthoxyéthoxy, méthylthiométhoxy, 2-méthylthioéthoxy, 2-éthylthioéthoxy, phénoxyméthoxy, 2-phénoxyéthoxy, alkylthio en $C_1$-$C_4$, trifluorométhylthio, dichlorofluorométhylthio, méthoxyméthylthio, 2-méthoxyéthylthio, éthoxyméthylthio, 2-éthoxyéthylthio, méthylthiométhylthio, amino, diméthylamino, diéthylamino, monométhylamino, monoéthylamino ou acétylamino,

Y représente CO ou $SO_2$,

n est un nombre entier égal à 0, 1, 2 ou 3,

$R^2$ représente l'hydrogène, un groupe alkyle saturé en $C_1$-$C_{12}$, un groupe halogénoalkyle contenant 1 à 4 atomes de carbone et 1 à 3 atomes identiques ou différents de fluor, de brome ou de chlore, un groupe hydroxyalkyle en $C_1$-$C_3$ alkoxyalkyle ou alkylthioalkyle contenant chacun 1 à 4 atomes de carbone dans les groupes alkyle, un groupe cycloalkyle en $C_3$-$C_7$ qui peut éventuellement porter 1 à 4 substituants méthyle, un

groupe phényle portant éventuellement 1 à 3 substituants identiques ou différents choisis parmi les groupes alkyle en $C_1$-$C_4$, le fluor, le chlore, le brome, les groupes nitro, trifluorométhyle, dichlorofluorométhyle, cyano, méthoxy, éthoxy, diméthylamino, diéthylamino, acétylamino, phényle, carbonyle, carboxamide ou ester d'acides carboxylique, ou encore le furanne, le tétrahydrofuranne ou le benzofuranne, qui peuvent éventuellement être substitués par des groupes méthyle, éthyle, le fluor, le chlore, des groupes cyano, phényle, amino substitué ou carboxyle, ou un groupe $OR^3$ ou encore $SR^3$ lorsque Y = CO, $R^3$ représentant un groupe alkyle en $C_1$-$C_6$, halogénoalkyle contenant 1 à 3 atomes de carbone et 1 à 5 atomes identiques ou différents de fluor ou de chlore, un groupe phényle portant éventuellement 1 à 3 substituants identiques ou différents choisi parmi les groupes méthyle, éthyle, le fluor, le chlore, le brome, les groupes alcoxy en $C_1$-$C_4$, di-(alkyle en $C_1$-$C_4$)-amino, nitro, cyano ou ester d'acide carboxylique,

$R^2$ représente $-N\langle^{R^4}_{R^5}$ dans lequel $R^4$ représente l'hydrogène ou un groupe alkyle en $C_1$-$C_4$ et $R^5$ représente l'hydrogène, un groupe alkyle en $C_1$-$C_6$, et

X représente =CH- ou un atome d'azote,

caractérisé en ce que l'on fait réagir une 1-hétéroaryl-4-aryl-pyrazoline-5-one de formule II ou IIA

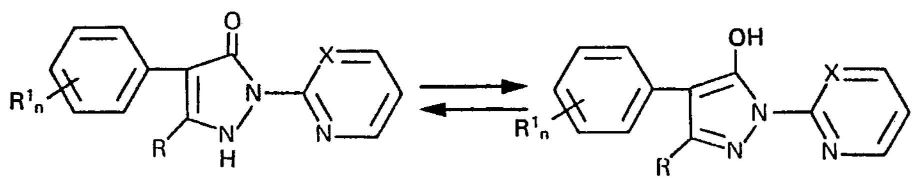

(II) (IIA)

dans laquelle

R, $R^1$, X et n ont les significations indiquées ci-dessus, avec un dérivé réactif d'un acide de formule III

$R^2$-Y-Z (II)

dans laquelle

$R^2$ et Y ont les significatins indiquées ci-dessus, et

Z représente un halogène, un groupe acyloxy, un groupe sulfonyloxy ou un radical d'azole, éventuellement en présence d'un capteur d'acide.

4. Produits microbicides caractérisés en ce qu'ils contiennent au moins un dérivé de 1-hétéroaryl-4-aryl-pyrazole de formule I ou IA selon les revendications 1 à 3.

5. Procédé pour combattre les microbes, caractérisé en ce que l'on fait agir sur les microbes et/ou leur habitat des dérivés de 1-hétéroaryl-4-aryl-pyrazoles de formule I ou IA selon les revendications 1 à 3.

6. Utilisation des dérivés de 1-hétéroaryl-4-arylpyrazoles de formules I ou IA selon les revendications 1 à 3 dans la lutte contre les microbes.

7. Procédé de préparation de produits microbicides, caractérisé en ce que l'on mélange des dérivés de 1-hétéroaryl-4-aryl-pyrazoles de formule I ou IA selon les revendications 1 à 3 avec des diluants et/ou des agents tensioactifs.